# EUROPEAN PATENT SPECIFICATION

(11) **EP 1 470 780 B1**
(45) Date of publication and mention of the grant of the patent: **11.04.2007**
(21) Application number: 04252327.4
(22) Date of filing: 21.04.2004
(51) Int. Cl.: A61B 5/00

(54) **Apparatus and method of operating apparatus for diagnosing sleep apnea**
Vorrichtung und Verfahren zur Schlafapnödiagnose
Dispositif et méthode pour le diagnostic de l'apnée du sommeil

(30) Priority: 25.04.2003 KR 2003026396
(43) Date of publication of application: 27.10.2004
(73) Proprietor: SAMSUNG ELECTRONICS CO., LTD., Suwon-city, Gyeonggi-do (KR)
(72) Inventor: Lee, Jong-youn, Giheung-eub Yonging-si Gyeonggi-do (KR); Yoon, Gil-won, Seongdong-gu Seoul (KR)
(74) Representative: Greene, Simon Kenneth

(56) References cited:
- EP-A- 0 335 356
- WO-A-99/63883
- US-A- 4 651 741
- US-B1- 6 172 743

## Description

The present invention relates to an apparatus for diagnosing sleep apnea, and more particularly, to an apparatus for detecting whether or not a temporary absence or cessation of breathing occurs during sleep by applying lights of two different wavelengths to some body part.

Sleep apnea is a temporary absence or cessation of breathing during sleep, and oxygen saturation represents the amount of oxygen that is contained in the blood. In general, when no oxygen enters the body due to sleep apnea, the oxygen saturation in the blood lowers to an abnormal level.

Sleep fragmentation at night due to sleep apnea causes excessive daytime sleepness (EDS) and decline in arterial oxygen saturation. Decline in oxygen saturation causes high blood pressure, arrythmia, or the like, and sometimes, even leads to fatal results, such as a heart attack during sleep and sudden death. It is reported that about 20 percent of America's adult population suffer from snoring, and 50 percent of the snorers suffer from sleep apnea.

In the meantime, children with sleep apnea show such symptoms as scatterbrained behavior, EDS, irregular sleep, rib cage retraction, and flaring of the ribs, and suffer from a poor academic record and mental or psychological disorders in serious cases. For infants or babies, sleep apnea can become a cause of sudden death during sleep.

Sleep apnea is typically classified into three main types: obstructive, central, and mixed. Among the three types, obstructive sleep apnea is featured by repeated closing of upper airway and results in a fall in arterial oxygen saturation. It is clinically classified into sleep apnea where a cessation of breathing for 10 seconds or more occurs five times in an hour or thirty times during 7 hour night sleep. Snoring is a sound made when a soft palate of an upper airway vibrates, and thus, is often a direct precursor of sleep apnea.

A sleep apnea test is generally carried out through polysomnography. Polysomnography is a test during which sleep architecture and function and behavioral events during sleep are objectively measured and recorded. To be specific, a number of physiological variables, such as brain waves, eye movement, chin electromyogram, leg electromyogram, electrocardiogram, snoring, blood pressure, breathing, and arterial oxygen saturation, are measured extensively, and at the same time, behavioral abnormality during sleep are recorded with video tape recorders. Trained technicians and sleep specialists read the record to obtain comprehensive results about the severity of snoring, whether arrythmia occurs, whether blood pressure increases, whether other problems are caused during sleep, and at what points the record is different from normal sleep patterns.

U.S. Patent No. 6,415,174 discloses "electrocardiogram (ECG)-derived respiratory rhythms for improved diagnosis of sleep apnea". The technology measures an ECG based on effects on an electrode attached to the chest, which expands due to respiration. The technology adopts signal processing of a cubic spline interpolation technique to extract information on the effect of respiration on ECG signals. This method is used to understand the correlation between respiration and ECG signals when respiration is difficult to be measured using another apparatus. However, this method has a disadvantage in that the electrode needs to be attached to a specific portion of the chest to measure the ECG, and accordingly, it is not easy to prepare such a complicated measurement process.

U.S. Patent No. 6,368,287 discloses "an integrated sleep apnea screening system", that enables general people to easily measure respiration at home without the help of clinicians or specialists. The system diagnoses sleep apnea by checking a temperature change from an inhaled and exhaled nasal air flow by means of a temperature sensor placed near the nostrils. The technology uses a light emitting diode (LED) to indicate that the sensor is correctly positioned. Since the temperature sensor placed near the nostrils cannot be used to diagnose central sleep apnea, respiration cannot be measured solely based on the temperature change of the air flow from the nose in a practical clinical diagnosis.

U.S. Patent No. 6,342,039 discloses "a microprocessor system for the simplified diagnosis of sleep apnea". The system diagnoses sleep apnea based on a phenomenon that oxygen saturation is restored to a normal level when normal breathing is established. The technology diagnoses sleep apnea by continuously measuring and graphing oxygen saturation levels and calculating the slope of an oxygen saturation graph which varies when sleep apnea occurs. To measure oxygen saturation, the technology performs a relatively complicated procedure of calculating peak and valley values of lights of two different wavelengths and building a regression equation using a ratio between the peak and valley values. However, a photo-plethysmography (PPG) waveform used to measure oxygen saturation of the human body is easily affected by parameters including motion artifact noise caused due to body movement, and the peak and valley value measurement is subject to error due to high frequency noise.

U.S. Patent No. 6,047,203 discloses "a physiologic sign feedback system", in which sensors are attached to a garment comprising a shirt as an integral part of the garment to measure respiration so that physiological signals of the human body, such as respiratory signals and electrocardiogram signals, can be easily monitored. An alarm informing abnormality is sent to a patient or a tester when an abnormal physiological signal is detected during a long test period. Although the disclosed garment-type system is manufactured for the patient's easy wear, the patient can feel uncomfortable with the system which is fastened to the person's body to measure respiration.

U.S. Patent No. 5,396,893 discloses "a method and apparatus for analyzing heart and respiratory frequencies photo-plethysmographically". The method diagnoses sleep apnea by analyzing PPG according to frequency bands and extracting respiration frequencies. When the method is used for normal people, a very exact prediction can be made. However, even when respiration does not occur, the system may determine that respiration has occurred due to ringing phenomena of a filter during the respiratory frequency extracting process.

According to an aspect of the present invention, there is provided an apparatus for diagnosing sleep apnea according to claim 1.

The present invention provides an apparatus for diagnosing sleep apnea by measuring photo-plethysmography (PPG) using two different wavelength lights and calculating a ratio between two measured values.

According to another aspect of the present invention, there is provided a method of operation according to claim 7.

The above and other features and advantages of the present invention will become more apparent by describing in detail exemplary embodiments thereof with reference to the attached drawings in which:
FIG. 1 is a block diagram of an apparatus for diagnosing sleep apnea according to the present invention;
FIG. 2 is a graph illustrating absorption coefficients of oxyhemoglobin and deoxyhemoglobin according to wavelengths;
FIG. 3 is a graph illustrating a typical waveform of photo-plethysmography (PPG);
FIG. 4 is a graph illustrating PPG waveforms according to various wavelengths during a normal breathing state;
FIG. 5 is a graph illustrating PPG waveforms according to various wavelengths during a breathing cessation state;
FIG. 6 is a graph illustrating a direct current (DC) component ration between lights of red wavelength range and an infrared wavelength range during a normal breathing state; and
FIG. 7 is a graph illustrating a DC component ratio between lights of a red wavelength range and an infrared wavelength range during a breathing cessation state.

The present invention will now be described more fully with reference to the accompanying drawings, in which preferred embodiments of the invention are shown.

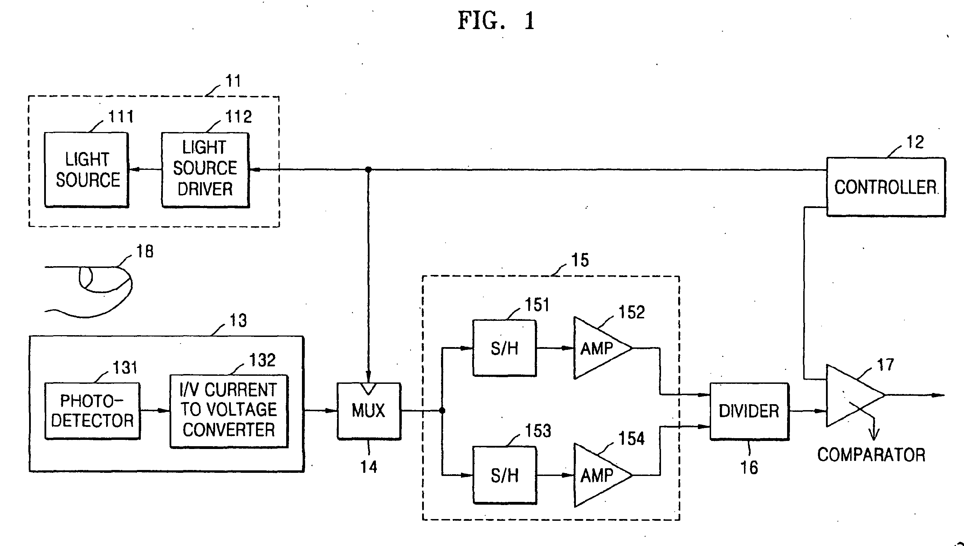

FIG. 1 is a block diagram of an apparatus for diagnosing sleep apnea according to the present invention. As shown in FIG. 1, the sleep apnea diagnosing apparatus includes a light source unit 11, a controller 12, a photo-detecting unit 13, a multiplexer (MUX) 14, a delay unit 15, a divider 16, and a comparator 17.

The light source unit 11 includes a light source 111 which generates light signals of different wavelength ranges and a light source driver 112 which provides driving currents to the light source 111.

The photo-detecting unit 13 includes a photo-detector 131 which converts optical signals generated by the light source 111 into currents and a current to voltage (I/V) converter 132 which converts the currents into voltages.

The delay unit 15 includes two sample-and-holders 151 and 153, which respectively sample signals output from the MUX 14 and delay the sampled signals for a predetermined period of time, and two amplifiers 152 and 154.

The MUX 14, the delay unit 15, the divider 16, and the comparator 17 process the voltages output from the photo-detecting unit 13 to determine the presence or absence of sleep apnea.

Operation of the sleep apnea diagnosing apparatus constructed as above will be explained. The light source driver 112 outputs driving currents to the light source 111 according to a control signal of the controller 12. The control signal indicates whether the light source 111 generates a red wavelength light or an infrared wavelength light. Here, the light source 111 is preferably a light emitting diode array comprising at least two light emitting diodes which emit lights of red and infrared wavelength ranges.

The controller 12 outputs the control signal to sequentially turn on and off the light emitting diode array according to the wavelengths.

The lights generated by the light source 111 are applied to some body part 18, such as a finger to measure an amount of oxygen in hemoglobin in the blood. Here, the body part 18 can be any body part where an arterial pulsating component can be measured.

The photo-detector 131 detects the lights which are generated by the light source 111 and pass through or are reflected on the body part 18, and outputs currents corresponding to the intensity of the detected lights. The IN converter 132 converts the currents into voltages.

The MUX 14 separates the lights output from the photo-detecting unit 13 into a red light and an infrared ray according to the wavelengths under the control of the control signal output from the controller 12.

The delay unit 15 samples voltages of the two lights which are separated by and output from the MUX 14 and holds for each predetermined period of time and amplifies the sampled voltages. This is done in order to properly delay the two lights separately output from the MUX 14 with a time difference therebetween so as to remove the difference and output the two lights substantially at the same time.

The divider 16 divides the two voltages output from the two amplifiers 152 and 154. The comparator 17 compares a reference value provided from the controller 12 with a resultant value obtained by the divider 16 to determine whether or not a subject is in sleep apnea. If it is determined that the subject is in sleep apnea, the controller 12 can send an alarm message to the subject or a tester using an additional alarm device (not shown). The comparator 17 and the controller 12 can be wirelessly connected, if necessary, so as not to restrict movement.

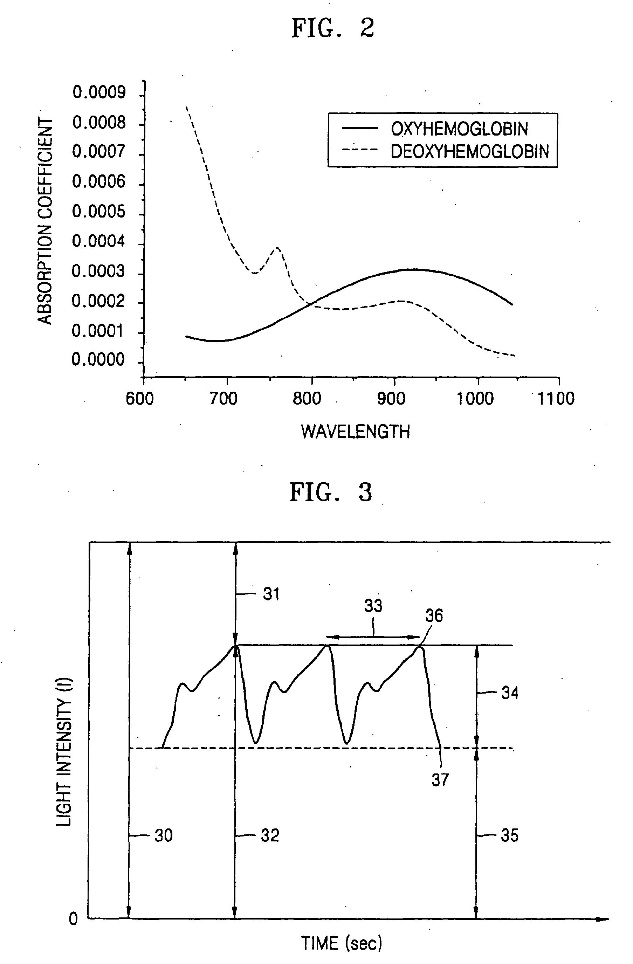

The theory on which the sleep apnea diagnosing apparatus is based will be explained as follows. When breathing temporarily stops, for example, due to sleep apnea, and thus no oxygen is entering the body, oxygen saturation in the blood abnormally decreases. Oxygen saturation can be optically measured. FIG. 2 is a graph illustrating absorption coefficients of oxyhemoglobin and deoxyhemoglobin according to wavelengths. As shown in FIG. 2, absorption coefficient curves with respect to wavelength can be differed entirely according to whether or not hemoglobin in the blood contains oxygen. Therefore, oxygen saturation can be predicted by comparing the amount of a reacted light of a red wavelength range in the body with the amount of a reacted light of an infrared wavelength range in the body. When lights are applied to a body part to predict the oxygen saturation levels, a photo-plethysmography (PPG) waveform as shown in FIG. 3 is obtained through transmission or reflection of the applied lights. In the waveform, reference numeral 30 denotes the quantity of a light applied to the body, reference numeral 31 denotes the quantity of an absorbed light, reference numeral 32 denotes the quantity of a light passing through the body, reference numeral 33 denotes a cardiac cycle, reference numeral 34 denotes the amount of change in the intensity of the transmitted lights due to an arterial pulsating component, namely, an alternating current (AC) component, reference numeral 35 denotes the amount of change in the intensity of the transmitted lights due to an arterial non-pulsating component, namely, a direct current (DC) component, reference numeral 36 denotes a peak of heartbeat, and reference numeral 37 denotes a valley of heartbeat.

The AC component in the PPG waveform is obtained by measuring a change in blood flow, which reflects a change of blood stream due to heartbeat. A method of measuring a heart rate using the AC component is widely known. The AC component may be generated by respiration or a person's voluntary or involuntary movement. At this time, the AC component is non-periodical and weaker in power than that generated due to a heartbeat. The DC component in the PPG waveform is generated when light is absorbed or scattered by buried objects, such as bones, skin, or hypodermis, which are not varied according to time.

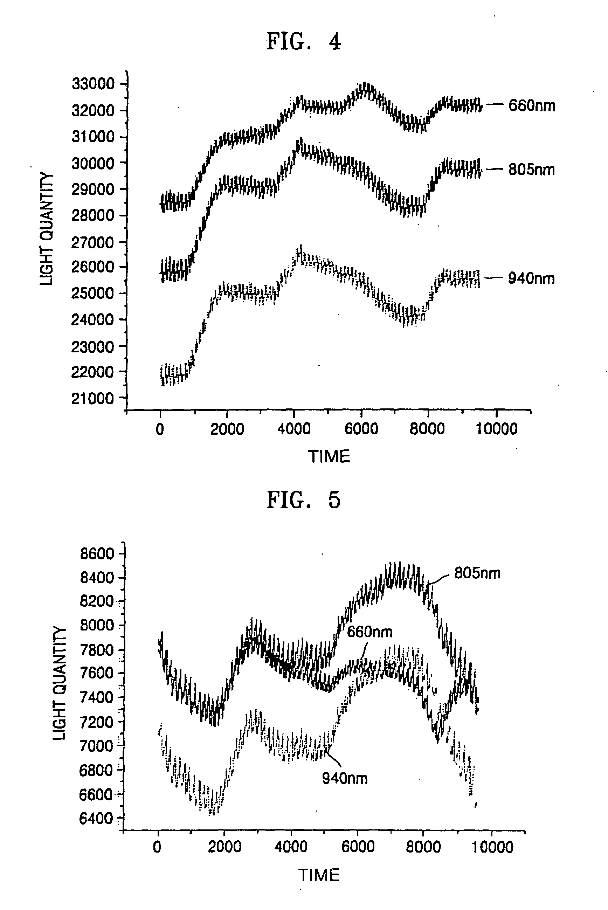

When no oxygen is entering the body because of sleep apnea, the amount of deoxyhemoglobin increases, and accordingly, a red wavelength light, which has a greater deoxyhemoglobin absorption coefficient than the one of an infrared light, gets more attenuated, as shown in FIG. 2. FIGS. 4 and 5 illustrate respectively PPG waveforms during a normal breathing state and a breathing cessation state. As shown in FIGS. 4 and 5, during a normal breathing state, similar slope patterns are shown over time. However, during a breathing cessation state, the amount of a light of a red wavelength of 660nm decreases due to the increase in the amount of deoxyhemoglobin.

In general, the DC component in the PPG waveform of FIG. 2 makes a great difference according to the thickness of a finger or characteristics of a pertinent body part through which a light transmits. Thus, if a transmission ratio between different wavelength lights is obtained, the difference made according to the characteristics of the pertinent body part can be compensated by the transmission ratio. The present invention uses DC components of a red light and an infrared light output from the MUX 14 and the delay unit 15. The divider 16 divides the DC component of the infrared light by the DC component of the red light. The value obtained by the division is referred to as a ratiometric index (RI). The comparator 17 compares the RI with the reference value provided by the controller 12, and determines that breathing has temporarily ceased during sleep when the output value of the divider 16 is greater than the reference value.

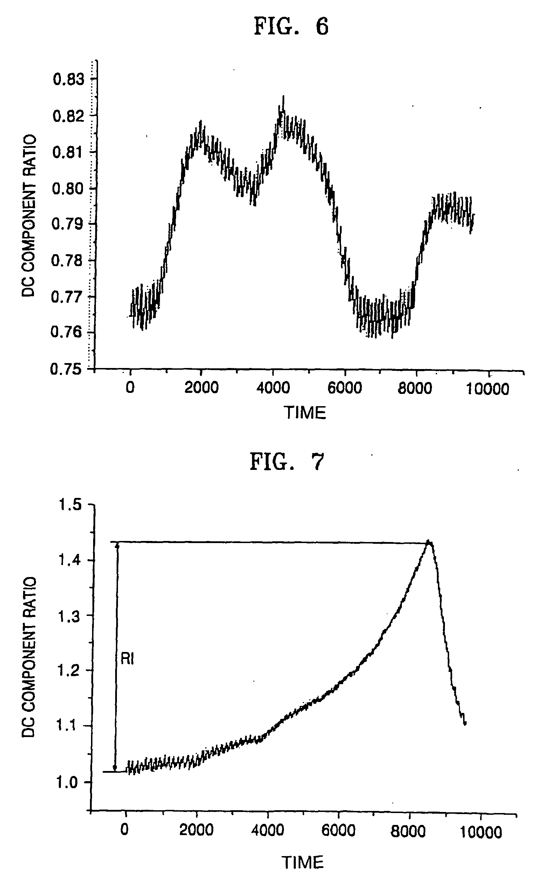

FIGS. 6 and 7 illustrate respectively a DC component ratio between a red wavelength light and an infrared wavelength light during a normal breathing state and a breathing cessation state. Referring to FIGS. 6 and 7, during a normal breathing state, the DC shows a similar shape to the original light. During a breathing cessation state, however, the DC component ratio between the two lights, i.e., RI, increases over time. As a consequence, when the RI is greater than the reference value, it is determined that sleep apnea occurs.

Table 1 shows measurement results of average Rls during a breathing cessation state and a normal breathing state. The measurements were repeatedly taken on six people six times per minute. For every minute measurement, the six people in the test were allowed to cease their breathing for 10 seconds or more three times and breathe normally three times. Two lights of a red wavelength of 660nm and an infrared wavelength of 940nm were utilized.

**[Table 1]**

| Testee | Average RI during breathing cessation state | Average RI during normal breathing state |
|---|---|---|
| 1 | 0.3±0.03 | 0.024±0.004 |
| 2 | 0.15±0.07 | 0.027±0.01 |
| 3 | 0.49±0.06 | 0.024±0.005 |
| 4 | 0.2±0.05 | 0.029±0.009 |
| 5 | 0.32±0.05 | 0.03±0.008 |
| 6 | 0.14±0.03 | 0.035±0.009 |

Here, an RI in data measured when breathing has stopped for 10 seconds or more ranges from 0.096 to 0.56. An RI in data measured when breathing is normal ranges from 0.02 to 0.07. It is preferable that a value in a range from 0.07 to 0.096 is adopted as the reference value of the comparator 17.

In the conventional art, when there is data where any peak value cannot be found in a PPG waveform affected by noise due to respiration or movement, the data is reflected in the prediction of oxygen saturation. When sleep apnea is diagnosed based on that predicted oxygen saturation, it results in errors.

Table 2 shows the difference between a predicted value of oxygen saturation and a measured value when the six people intentionally made noise three times by moving their bodies.

**[Table 2]**

| People in test | Average error (%) |
|---|---|
| 1 | 9.7±1.2 |
| 2 | 5.7±0.6 |
| 3 | 6±0.06 |
| 4 | 1.3±0.6 |
| 5 | 3±0.5 |
| 6 | 3.7±0.6 |

According to Table 2, since the present invention uses the DC component ratio instead of peak and valley values in the AC component, it is not affected by noise due to movement when sleep apnea is diagnosed.

As described above, the present invention can diagnose sleep apnea at home irrespective of the causes of the sleep apnea. Furthermore, the present invention can measure the RI without being affected by noise from voluntary or involuntary movement generated during sleep, differently from the conventional art.

Reflection- or transmission-type PPG waveforms are measured with relative ease, such that they can be measured using any body part, such as a finger, toe, wrist, and the crown of an infant's head.

Since a conventional breathing cessation detector or an impedance change detector employing a method of measuring oxygen saturation levels uses an analogue to digital (A/D) converter, it requires a high performance microcontroller. However, since the present invention uses analogue hardware without an A/D converter, it can be realized in a low performance microcontroller.

The conventional PPG for measuring oxygen saturation needs to detect both peak and valley values of an AC component. In this process, errors can occur in the detected results because of internal and external parameters including a person's movement and respiration. However, the present invention uses the DC value in the PPG, such that it is relatively less prone to noise, and DC varying factors of low frequency can be eliminated by measurement of a ratio between two lights.

While the present invention has been particularly shown and described with reference to exemplary embodiments thereof, it will be understood by those of ordinary skill in the art that various changes in form and details may be made therein without departing from the scope of the present invention as defined by the following claims.

## Claims

1. An apparatus for diagnosing sleep apnea, which detects a temporary cessation of breathing during sleep by applying lights to a body part and processing output lights as DC components, the apparatus comprising:
a light source unit (11) which is arranged to sequentially generate light signals of at least two different wavelengths according to a predetermined control signal;
a photo-detecting unit (13) which is arranged to detect the light signals, which are generated by the light source unit and then applied to the body part (18), and to convert the detected light signals into electric signals;
a diagnosis unit (14, 15, 16, 17) which is arranged to remove a time delay between the electric signals output from the photo-detecting unit (13) substantially, to calculate a ratio between the electric signals, and to compare the ratio with a predetermined reference value to diagnose sleep apnea; and
a controller (12) which is arranged to output the control signal to the light source unit to generate at least two different wavelength light signals, and to provide the reference value to the diagnosis unit:
**characterised in that** of the ratio is the DC component of one of the detected light signals divided by the DC component of another one of the detected light signals at a different wavelength.

2. The apparatus of claim 1, wherein the light source (11) unit is a light emitting diode array which generates lights of at least a red wavelength range and an infrared wavelength range.

3. The apparatus of claim 2, wherein the light source unit (11) is arranged to apply the generated lights to a body part where an arterial pulsating component is measured.

4. The apparatus of claim 2 or 3, wherein the controller (12) outputs the control signal to the light source unit (11) to sequentially turn on and off the light emitting diode array according to the wavelengths.

5. The apparatus of any preceding claim, wherein the photo-detecting unit (13) includes:
a photo-detector (131) which detects the lights, which are generated by the light source unit and applied to the body part (18), and outputs current signals; and
a current to voltage converter (132) which converts the current signals into voltage signals.

6. The apparatus of any preceding claim, wherein the diagnosis unit includes:
a multiplexer (14) which separates the electric signals output from the photo-detecting unit according to the control signal;
a delay unit (15) which respectively samples the separated electric signals and delays the sampled electric signals for a period of time to output the sampled electric signals at the substantially same time;
a divider (16) which calculates a ratio of the sampled electric signals output from the delay unit; and
a comparator (17) which compares the calculated ratio with the reference value to determine the presence or absence of sleep apnea.

7. A method of operation of an apparatus for diagnosing sleep apnea including a divider (16) and comparator (17), the method comprising:
(a) sequentially generating light signals of at least two different wavelengths;
(b) detecting the light signals, which are generated in step (a) and applied to the body part, and converting the light signals into electric signals;
(c) sampling the electric signals output from step (b) and respectively delaying the electric signals in order to remove the difference substantially between the sampled electric signals; and
(d) calculating by means of the divider (16) a ratio of the electrical signals and comparing by means of the comparator (17) the calculated ratio with a predetermined reference value to determine the presence or absence of sleep apnea, of **characterised in that** the ratio is the DC component of one of the detected light signals divided by the DC component of another one of the detected light signals at a different wavelength..

8. The method of claim 7, wherein the two wavelength ranges in step (a) are a red wavelength range and an infrared wavelength range.

9. The method of claim 7 or 8, wherein the lights generated in step (a) 5 can be applied to a body part (18) where an arterial pulsating component is measured.

## Patentansprüche

1. Vorrichtung zur Diagnose von Schlafapnoe, die ein temporäres Aussetzen der Atmung während des Schlafs erfasst, indem Licht auf einen Körperteil eingestrahlt wird und Ausgangslicht als DC-Komponenten verarbeitet wird, wobei die Vorrichtung umfasst:
eine Lichtquelleneinheit (11), die so ausgebildet ist, dass sie, entsprechend einem bestimmten Steuersignal sequentiell Lichtsignale mit mindestens zwei verschiedenen Wellenlängen erzeugt;
eine Photodetektoreinheit (13), die so ausgebildet ist, dass sie die Lichtsignale erfasst, die von der Lichtquelleneinheit erzeugt und dann auf den Körperteil (18) eingestrahlt sind, und die erfassten Lichtsignale in elektrische Signale konvertiert;
eine Diagnoseeinheit (14, 15, 16, 17), die so ausgebildet ist, dass sie eine Zeitverzögerung zwischen den elektrischen Signalen, die von der Photodetektoreinheit (13) ausgegeben sind, im Wesentlichen eliminiert, ein Verhältnis zwischen den elektrischen Signalen berechnet und das Verhältnis mit einem bestimmten Referenzwert vergleicht, um Schlafapnoe zu diagnostizieren; und
eine Steuerung (12), die so ausgebildet ist, dass sie das Steuersignal an die Lichtquelleneinheit ausgibt, so dass Lichtsignale mit mindestens zwei verschiedenen Wellenlängen erzeugt werden, und den Referenzwert an die Diagnoseeinheit gibt:
**dadurch gekennzeichnet, dass** das Verhältnis die DC-Komponente eines der erfassten Lichtsignale geteilt durch die DC-Komponente eines anderen der erfassten Lichtsignale bei einer anderen Wellenlänge ist.

2. Vorrichtung nach Anspruch 1, wobei die Lichtquelleneinheit (11) ein LED-Array ist, das Licht mindestens in einem roten Wellenlängenbereich und in einem infraroten Wellenlängenbereich erzeugt.

3. Vorrichtung nach Anspruch 2, wobei die Lichtquelleneinheit (11) so ausgebildet ist, dass sie erzeugtes Licht auf einen Körperteil einstrahlt, an dem eine arterielle Pulskomponente gemessen wird.

4. Vorrichtung nach Anspruch 2 oder 3, wobei die Steuerung (12) das Steuersignal an die Lichtquelleneinheit (11) ausgibt, so dass das LED-Array entsprechend den Wellenlängen sequentiell an und aus geschaltet wird.

5. Vorrichtung nach einem der vorhergehenden Ansprüche, wobei die Photodetektoreinheit (13) aufweist:
einen Photodetektor (131), der das Licht erfasst, das von der Lichtquelleneinheit erzeugt und auf den Körperteil (18) eingestrahlt ist, und Stromsignale ausgibt; und
einen Strom-Spannungs-Konverter (132), der die Stromsignale in Spannungssignale umwandelt.

6. Vorrichtung nach einem der vorhergehenden Ansprüche, wobei die Diagnoseeinheit aufweist:
einen Multiplexer (14), der entsprechend dem Steuersignal die von der Photodetektoreinheit ausgegebenen elektrischen Signale trennt;
eine Verzögerungseinheit (15), die jeweils die getrennten elektrischen Signale abtastet und die abgetasteten elektrischen Signale um eine Zeitspanne verzögert, so dass die abgetasteten elektrischen Signale im Wesentlichen zur selben Zeit ausgegeben werden;
einen Teiler (16), der ein Verhältnis der abgetasteten elektrischen Signale berechnet, die von der Verzögerungseinheit ausgegeben sind; und
einen Komparator (17), der das berechnete Verhältnis mit dem Referenzwert vergleicht, um das Vorliegen oder Fehlen von Schlafapnoe zu bestimmen.

7. Verfahren zum Betrieb einer Vorrichtung zur Diagnose von Schlafapnoe mit einem Teiler (16) und einem Komparator (17), wobei das Verfahren umfasst:
(a) sequentielles Erzeugen von Lichtsignalen mit mindestens zwei verschiedenen Wellenlängen;
(b) Erfassen der Lichtsignale, die in Schritt (a) erzeugt und auf den Körperteil eingestrahlt sind, und Konvertieren der Lichtsignale in elektrische Signale;
(c) Abtasten der in Schritt (b) ausgegebenen elektrischen Signale und entsprechendes Verzögern der elektrischen Signale, um die Differenz zwischen den abgetasteten elektrischen Signalen im Wesentlichen zu eliminieren; und
(d) Berechnen eines Verhältnisses der elektrischen Signale mittels des Teilers (16) und Vergleichen des berechneten Verhältnisses mit einem bestimmten Referenzwert mittels des Komparators (17), um das Vorliegen oder Fehlen von Schlafapnoe zu bestimmen, **dadurch gekennzeichnet, dass** das Verhältnis die DC-Komponente eines der erfassten Lichtsignale geteilt durch die DC-Komponente eines anderen der erfassten Lichtsignale bei einer anderen Wellenlänge ist.

8. Verfahren nach Anspruch 7, wobei die beiden Wellenlängenbereiche in Schritt (a) ein roter Wellenlängenbereich und ein infraroter Wellenlängenbereich sind.

9. Verfahren nach Anspruch 7 oder 8, wobei das in Schritt (a) erzeugte Licht auf einen Körperteil (18) eingestrahlt werden kann, an dem eine arterielle Pulskomponente gemessen wird.

## Revendications

1. Appareil pour diagnostiquer une apnée du sommeil, qui détecte un arrêt temporaire de la respiration pendant le sommeil en appliquant des lumières à une partie de corps et en traitant les lumières émises comme des composantes de courant continu, l'appareil comprenant :
une unité de source de lumière (11) agencée pour générer séquentiellement des signaux lumineux d'au moins deux longueurs d'onde différentes selon un signal de commande prédéterminé ;
une unité de photodétection (13) agencée pour détecter les signaux lumineux générés par l'unité de source de lumière et appliqués ensuite à la partie de corps (18) et pour convertir les signaux lumineux détectés en signaux électriques;
une unité de diagnostic (14, 15, 16, 17) agencée pour supprimer essentiellement un retard de temps entre les signaux électriques émis par l'unité de photodétection (13), calculer un rapport entre les signaux électriques et comparer le rapport avec une valeur de référence prédéterminée pour diagnostiquer une apnée du sommeil ; et
un dispositif de commande (12) agencé pour émettre le signal de commande à l'unité de source de lumière pour générer au moins deux signaux lumineux de longueur d'onde différente, et pour transmettre la valeur de référence à l'unité de diagnostic ;
**caractérisé en ce que** le rapport est la composante de courant continu de l'un des signaux lumineux détectés divisée par la composante de courant continu d'un autre des signaux lumineux détectés à une longueur d'onde différente.

2. Appareil selon la revendication 1, dans lequel l'unité de source de lumière (11) est un réseau de diodes électroluminescentes générant des lumières d'au moins une plage de longueur d'onde rouge et une plage de longueur d'onde infrarouge.

3. Appareil selon la revendication 2, dans lequel l'unité de source de lumière (11) est agencée de manière à appliquer les lumières générées à une partie de corps où une composante de pouls artériel est mesurée.

4. Appareil selon la revendication 2 ou 3, dans lequel le dispositif de commande (12) émet le signal de commande à l'unité de source de lumière (11) afin d'activer et de désactiver séquentiellement le réseau de diodes électroluminescentes selon les longueurs d'onde.

5. Appareil selon l'une quelconque des revendications précédentes, dans lequel l'unité de photodétection (13) comprend :
un photodétecteur (131) qui détecte les lumières générées par l'unité de source de lumière et appliquées à la partie de corps (18) et qui émet des signaux de courant ; et
un convertisseur de courant - tension (132) qui convertit les signaux de courant en signaux de tension.

6. Appareil selon l'une quelconque des revendications précédentes, dans lequel l'unité de diagnostic comprend :
un multiplexeur (14) qui sépare les signaux électriques émis par l'unité de photodétection selon le signal de commande ;
une unité de retard (15) qui échantillonne respectivement les signaux électriques séparés et qui retarde les signaux électriques échantillonnés pendant une période de temps afin d'émettre les signaux électriques échantillonnés sensiblement en même temps ;
un diviseur (16) qui calcule un rapport des signaux électriques échantillonnés émis par l'unité de retard ; et
un comparateur (17) qui compare le rapport calculé avec la valeur de référence en vue de déterminer la présence ou l'absence d'une apnée du sommeil.

7. Procédé de commande d'un appareil pour diagnostiquer une apnée du sommeil, comprenant un diviseur (16) et un comparateur (17), le procédé comprenant :
(a) la génération séquentielle de signaux lumineux d'au moins deux longueurs d'onde différentes ;
(b) la détection des signaux lumineux générés à l'étape (a) et appliqués à la partie de corps, et la conversion des signaux lumineux en signaux électriques ;
(c) l'échantillonnage des signaux électriques émis à l'étape (b) et le retardement respectif des signaux électriques en vue de supprimer essentiellement la différence entre les signaux électriques échantillonnés ; et
(d) le calcul, au moyen du diviseur (16), d'un rapport des signaux électriques et la comparaison, au moyen du comparateur (17), du rapport calculé avec une valeur de référence prédéterminée en vue de déterminer la présence ou l'absence d'une apnée du sommeil ;
**caractérisé en ce que** le rapport est la composante de courant continu de l'un des signaux lumineux détectés divisée par la composante de courant continu d'un autre des signaux lumineux détectés à une longueur d'onde différente.

8. Procédé selon la revendication 7, dans lequel les deux plages de longueur d'onde à l'étape (a) sont une plage de longueur d'onde rouge et une plage de longueur d'onde infrarouge.

9. Procédé selon la revendication 7 ou 8, dans lequel les lumières générées à l'étape (a) peuvent être appliquées à une partie de corps (18) où une composante de pouls artériel est mesurée.
